(19) Europäisches Patentamt
European Patent Office
Office européen des brevets

(11) Veröffentlichungsnummer: **0 254 902 B1**

(12) **EUROPÄISCHE PATENTSCHRIFT**

(45) Veröffentlichungstag der Patentschrift: **28.08.91**

(51) Int. Cl.5: **A61K 31/675**, A61K 47/10

(21) Anmeldenummer: **87109559.2**

(22) Anmeldetag: **03.07.87**

Die Akte enthält technische Angaben, die nach dem Eingang der Anmeldung eingereicht wurden und die nicht in dieser Patentschrift enthalten sind.

# (54) Lösungen von Oxazaphosphorinen mit verbesserter Stabilität und Verfahren zu deren Herstellung.

(30) Priorität: **11.07.86 DE 3623369**

(43) Veröffentlichungstag der Anmeldung:
**03.02.88 Patentblatt 88/05**

(45) Bekanntmachung des Hinweises auf die Patenterteilung:
**28.08.91 Patentblatt 91/35**

(84) Benannte Vertragsstaaten:
**AT BE CH DE ES FR GB GR IT LI LU NL SE**

(56) Entgegenhaltungen:
**EP-A- 0 215 300**

**ZIEKENHUISFARMACIE, Band 1, Nr. 2, Seiten 57,58, 1985, NL; E.B.L.M. VAN NISPEN TOT PANNERDEN et al.: "Cyclofosfamide in oplossing"**

**MARTINDALE, The Extra Pharmacopeia, 28th edition, page 199**

(73) Patentinhaber: **ASTA Pharma Aktiengesellschaft**
**Weismüllerstrasse 45**
**W-6000 Frankfurt am Main 1(DE)**

(72) Erfinder: **Sauerbier, Dieter**
**Rauhe Horst 18**
**W-4806 Werther(DE)**
Erfinder: **Molge, Klaus, Dr.**
**Erfurter Strasse 9**
**W-4800 Bielefeld 14(DE)**
Erfinder: **Weigert, Werner, Dr.**
**Rhedaerstrasse 3**
**W-4800 Bielefeld 14(DE)**
Erfinder: **Isaac, Otto, Dr.**
**Liesingstrasse 8**
**W-6450 Hanau 9(DE)**

**EP 0 254 902 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Oxazaphosphorine gehören zur Gruppe der alkylierenden Cytostatika und werden zur Behandlung von Tumor-Erkrankungen eingesetzt.

Therapeutisch bedeutende Oxazaphosphorine, für die das erfindungsgemäße Verfahren beziehungsweise die entsprechenden Lösungen insbesondere in Frage kommen, sind zum Beispiel Cyclophosphamid und Ifosfamid. Die chemische Bezeichnung für Cyclophosphamid ist 2-[Bis-(2-chlorethyl)-amino]tetra-hydro-2H-1,3,2-oxazaphosphorin-2-oxid, für Ifosfamid 2-(Chlorethylamino)-3-(2-chlorethyl)-tetrahydro-2H-1,3,2-oxazaphosphorin-2-oxid.

Die therapeutische Anwendung von Cyclophosphamid und Ifosfamid erfolgt durch perorale und parenterale Zubereitungsformen. Der überwiegende Teil der parenteralen Applikation erfolgt in Form von Infusionen. Infolge der begrenzten Stabilität der Oxazaphosphorine in wäßriger Lösung sind lagerfähige gebrauchsfertige Injektionslösungen nicht bekannt. Parenterale Zubereitungsformen bestehen derzeit vielmehr aus Injektionsflaschen, die unterschiedliche Wirkstoffdosen als Trockensubstanz enthalten, aus denen die Injektionslösung unmittelbar vor der Applikation hergestellt wird.

Im Falle des Cyclophosphamids besteht die Trockensubstanz für die parenterale Anwendung beispielsweise aus einer keimfreien Mischung von kristallinem Cyclophosphamid-Monohydrat und Kochsalz. Der Einsatz einer derartigen keimfreien Kristallmischung ist gegenüber einer fertigen Injektionslösung jedoch mit erheblichen Nachteilen verbunden. So muß bereits bei der Gewinnung des Wirkstoffs beispielsweise bei der Kristallisation, beim Abzentrifugieren der Kristalle, beim Trocknen, Mahlen und Mischen mit dem ebenfalls aufwendig herzustellenden sterilen Kochsalz, steril und unter aseptischen Bedingungen gearbeitet werden. Schließlich muß das fertige Gemisch unter sterilen Kautelen abgefüllt werden. Dieses aufwendige Verfahren ist ständig der Gefahr partikulärer und mikrobieller Kontaminationen ausgesetzt.

Darüberhinaus läßt sich bei aller Sorgfalt in der Herstellung die Bildung verschieden großer Kristalle nicht vermeiden, so daß die Anfertigung von Cyclophosphamid-Lösungen mit Trockensubstanz in Injektionsflaschen einen erheblichen Zeitaufwand erfordert. Vor allem in Krankenanstalten führt dies zu einer häufig nicht zumutbaren Mehrbelastung des Krankenpflegepersonals.

Weiterhin ist die berufliche Exposition des Pflegepersonals bei der Anfertigung von Lösungen potentiell cancerogener Cytostatika zu beachten und eine Kontamination des Pflegepersonals sollte deshalb soweit wie möglich vermieden werden. Bei der Herstellung von Lösungen aus Trockensubstanz kann jedoch eine Einatmung derartiger Wirkstoffpartikel nicht mit Sicherheit ausgeschlossen werden.

Aus den vorgenannten Gründen besteht (insbesondere auch in Kliniken) das Bedürfnis nach einer einfach zu handhabenden Oxazaphosphorin-Formulierung zur parenteralen Applikation. Eine solche Zubereitungsform sollte eine Gefährdung des Plegepersonals soweit wie möglich ausschließen und nicht zuletzt eine kostengünstige Therapie erlauben.

Die Herstellung direkt injizierbarer stabiler wäßriger oder wasserhaltiger Oxazaphosphorinlösungen ist jedoch wegen der Instabilität der Oxazaphosphorine infolge hydrolytischer Zersetzung nicht möglich.

Eine 50 %ige (G/G) Lösung von Cyclophosphamid in 96 %igem (V/V) Ethanol ist bekannt (Ziekenhuisfarmacie, 1985, Bd. 1, Nr. 2, Seiten 57 - 58; Martindale, The Extra Pharmacopoeia 28. Auflage, Seite 199, linke Spalte). Jedoch geben diese Stellen keinen Hinweis für die technische Lehre, daß Lösungen von Oxazaphosphorinen in hochprozentigem Ethanol eine lägerfähige Form für solche Oxazaphosphorine darstellen, die eine verbesserte Anwendung in der Praxis ermöglicht.

Die Erfindung betrifft Lösungen von Oxazaphosphorinen der allgemeinen Formel

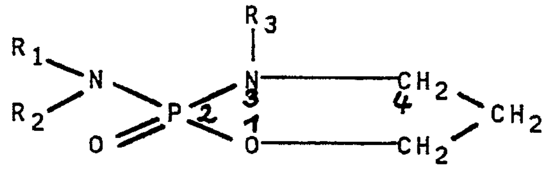

worin $R_1$, $R_2$ und $R_3$ gleich oder verschieden sind und Wasserstoff, Methyl, Ethyl, 2-Chlorethyl oder 2-Methansulfonyloxyethyl bedeuten und dabei mindestens zwei dieser Reste 2-Chlorethyl und/oder 2-Methansulfonyloxyethyl sind, in 80 - 100 %igem (V/V) *Ethanol, wobei die Oxazaphosphorin-Konzentration

* Prozent V/V (Prozentgehalt Volumen in Volumen) bedeuten die Anzahl Milliliter einer Substanz in 100 Milliliter Endprodukt.

10 - 70 % (G/V) ** beträgt, ausgenommen eine 50 %ige (G/G) Lösung von Cyclophosphamid in 96 %igem (V/V) Ethanol. Überraschenderweise weisen solche Lösungen eine ausgezeichnete Stabilität auf und sind daher für längere Zeit lagerfähig.

Als Oxazaphosphorin kommt insbesondere der Wirkstoff Cyclophosphamid oder Ifosfamid in Frage.

Die erfindungsgemäßen Lösungen können dann für die parenterale Applikation auf einfache Weise mit Wasser, Ringer-Lösung oder ähnlichen Infusionsflüssigkeiten verdünnt werden, wobei im allgemeinen die Verdünnung so erfolgen soll, daß die maximale Ethanolkonzentration nicht über 10 % liegt. Die Oxazaphosphorine werden überwiegend durch Infusion appliziert. Die erfindungsgemäßen Lösungen sind daher gerade als Infusionszusätze geeignet. Sie können beispielsweise direkt aus der Ampulle in die Infusionslösung überführt werden.

Die erfindungsgemäßen Lösungen besitzen daher im Vergleich zu Lösungen, die unmittelbar vor der Anwendung aus Sterilpulvern oder Lyophilisaten zubereitet werden, eine Reihe von Vorteilen:

- Sie sind weniger der Gefahr einer partikulären oder mikrobiellen Kontamination ausgesetzt (da zum Beispiel unmittelbar vor der Abfüllung filtriert werden kann).
- Sie lassen eine einfache Entnahme aliquoter Mengen zu, wobei der Wirkstoffgehalt stets einheitlich ist (bei einer Trockenabfüllung liegen stets Schwankungen hinsichtlich des Wirkstoffes vor).
- Sie machen den Lösungsvorgang überflüssig und sind unmittelbar gebrauchsfertig.
- Sie tragen zur Sicherheit des Pflegepersonals bei.
- Sie sind kostengünstiger in der Herstellung.

Die Erfindung betrifft weiterhin ein Verfahren zur Herstellung von Lösungen von Oxazaphosphorinen der allgemeinen Formel

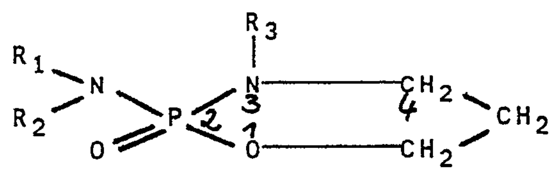

worin $R_1$, $R_2$ und $R_3$ gleich oder verschieden sind und Wasserstoff, Methyl, Ethyl , 2-Chlorethyl oder 2-Methansulfonyloxyethyl bedeuten, in 80 - 100 %igem (V/V) Ethanol, wobei die Oxazaphosphorin-Konzentration 10 - 70 % (G/V) beträgt, ausgenommen eine 50 %ige (G/G) Lösung von Cyclophosphamid in 96 %igem (V/V) Ethanol, indem man Oxazaphosphorine der Formel I bei einer Temperatur zwischen 15 und 40 °C in 80 - 100 %igem (V/V) wässrigen Ethanol, gegebenenfalls unter Rühren oder Bewegung des Lösungsmittels, auflöst, wobei auf 100 ml des Lösungsmittels 10 bis 150 g vorzugsweise 20 bis 80 g, insbesondere 30 bis 40 g Oxazaphophorin verwendet werden.

Selbstverständlich können die Oxazaphosphorine auch in Form von Lyophilisaten für die Herstellung der Lösungen verwendet werden.

Es ist auch möglich, zum Beispiel das Oxazaphosphorin in absolutem Ethanol oder auch 96 %igem Ethanol aufzulösen und dann durch Zusatz von Wasser eine niedrigere Ethanolkonzentration einzustellen. Das Auflösen erfolgt bei Temperaturen zwischen 15 und 40 °C, vorzugsweise 25, insbesondere 20 °C. Die Konzentration des in den erfindungsgemäßen Lösungen eingesetzten Ethanols ist 80 - 100 %, vorzugsweise 90 - 100 %, insbesondere 94 - 98 % (V/V).

Die Konzentration des Oxazaphosphorins in den erfindungsgemäßen Lösungen beträgt im allgemeinen 10 - 70 % (G/V), vorzugsweise 15 - 50 % (G/V), insbesondere 20 - 30 % (G/V). Für das Cyclophosphamid kommen beispielsweise folgende Konzentrationen in Frage:

10 - 60 % (G/V), vorzugsweise 15 - 40 % (G/V), insbesondere 20 - 30 % (G/V); Für Ifosfamid beispielsweise 10 - 70 % (G/V), vorzugsweise 15 - 50 % (G/V), insbesondere 20 - 30 % (G/V). Die erfindungsgemäßen Lösungen können auch 2 oder mehr verschiedene Oxazaphosphorin-Wirkstoffe enthalten. In solchen Fällen ist beispielsweise die Konzentration der Summe der Wirkstoffe 10 - 70, vorzugsweise 15 - 50, insbesondere 20 - 30 % (G/V).

Zum Auflösen werden die Oxazaphosphorine vorzugsweise in kristalliner Form (zum Beispiel feinkristalliner Form) eingesetzt. Selbstverständlich können für die Auflösung aber auch amorphe, halbfeste oder ölige Formen der Oxazaphosphorine verwendet werden. Das Auflösen kann gegebenenfalls unter Rühren

** Prozent G/V (Prozentgehalt Gewicht in Volumen) bedeutet die Anzahl Gramm einer Substanz in 100 Milliliter Endprodukt.

oder sonstiger Bewegung des Lösungsmittels erfolgen.

In den erfindungsgemäßen Lösungen erfolgt bei einer Lagerung bei 4° C während eines Jahres praktisch keine Zersetzung beziehungsweise eine solche ist nicht höher als 2 %. Selbst bei einer Lagertemperatur von 30° C bleiben die Lösungen klar und farblos und ohne Niederschlagsbildung.

Versuche mit anderen physiologisch verträglichen Lösungsmitteln, wie Glykofurol, Polyethylenglykol 300, Polyethylenglykol 400, 1,2-Propylenglykol, 1,3-Butylenglykol ergaben, daß die Wirkstoffe in diesen Mitteln trotz fehlenden Wassergehalts ganz erheblich instabiler waren, daß Verfärbungen auftraten und solche Lösungen als lagerfähige pharmazeutische Zubereitungen nicht verwendbar sind.

Die erhebliche verbesserte Haltbarkeit der erfindungsgemäß hergestellten ethanolischen Lösungen gegenüber beispielsweise wässrigen Lösungen geht aus der folgenden Tabelle hervor:

| Jährliche Zersetzungsrate | | in Wasser | in Ethanol 96%ig |
|---|---|---|---|
| Cyclophosphamid bei | 4°C | 25 % | 1,5 % |
| Cyclophosphamid bei | 20°C | 97 % | 15 % |
| Ifosfamid bei | 4°C | 2 % | 0,02 % |
| Ifosfamid bei | 20°C | 20 % | 0,3 % |

Beispiel 1

Herstellung einer 25 %igen Cyclophosphamid-Lösung

In einem geeigneten Behälter werden 6 Liter Ethanol 96 % DAB 8 * vorgelegt und unter Rühren 2,673 kg Cyclophosphamid-Monohydrat zugegeben. Nach Auflösen des Wirkstoffes - der Lösevorgang dauert nur wenige Minuten - wird mit Ethanol (96 %ig) auf 10 Liter (9,181 kg) aufgefüllt.

1 ml dieser Lösung enthält 250 mg Cyclophosphamid wasserfrei.

Zur Herstellung von Ampullen mit 4 ml Lösung wird die zuvor hergestellte Lösung unter aseptischen Bedingungen über ein Membranfilter 0,22 um (Filtermaterial Teflon) sterilfiltriert und in bekannter Weise unter Stickstoffbegasung zu 4 ml in farblose 5-ml-Ampullen dosiert.

1 Ampulle enthält 1 g Cyclophosphamid wasserfrei.

Beispiel 2

Herstellung einer 25 %igen Ifosfamid-Lösung

In einem geeigneten Behälter werden 6 Liter Ethanol 96 % DAB 8 vorgelegt und 2,5 kg Ifosfamid unter Rühren zugegeben. Nach wenigen Minuten hat sich der Wirkstoff aufgelöst. Schließlich wird mit 96 %igem Ethanol auf 10 Liter (9,025 kg) aufgefüllt. 1 ml Lösung enthält 250 mg Ifosfamid.

Zur Herstellung von Ampullen mit 8 ml Lösung Wird unter aseptischen Bedingungen über ein Membranfilter 0,22 um (Filtermaterial Teflon) sterilfiltriert und unter Stickstoffbegasung zu 8 ml in farblose 10-ml-Ampullen abgefüllt. Eine Ampulle enthält 2 g Ifosfamid.

## Patentansprüche

1. Lösungen von Oxazaphosphorinen der allgemeinen Formel

* DAB 8 = Deutsches Arzneibuch, 8. Auflage

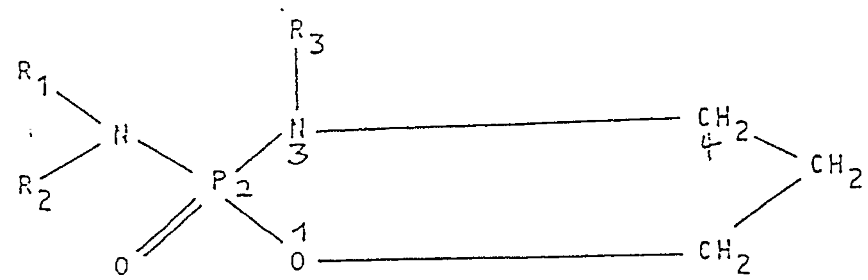

worin $R_1$, $R_2$ und $R_3$ gleich oder verschieden sind und Wasserstoff, Methyl, Ethyl, 2-Chlorethyl oder 2-Methansulfonyloxyethyl bedeuten und dabei mindestens zwei dieser Reste 2-Chlorethyl und/oder 2-Methansulfonyloxyethyl sind, in 80 - 100 %igem (V/V) *Ethanol, wobei die Oxazaphosphorin-Konzentration 10 - 70 Prozent (G/V) **beträgt, ausgenommen eine 50 %ige (G/G) Lösung von Cyclophosphamid in 96 %igem (V/V) Ethanol.

2. Verfahren zur Herstellung von Lösungen von Oxazaphosphorinen der allgemeinen Formel

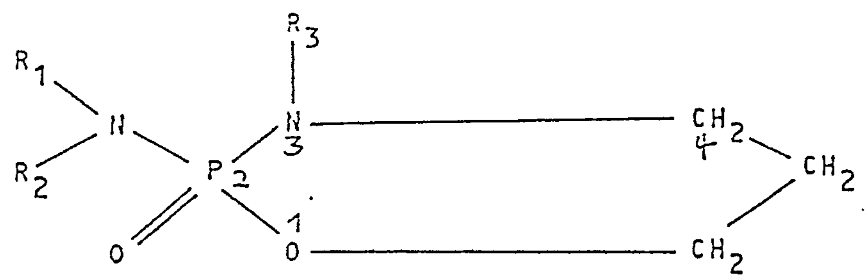

worin $R_1$, $R_2$ und $R_3$ gleich oder verschieden sind und Wasserstoff, Methyl, Ethyl, 2-Chlorethyl oder 2-Methansulfonyloxyethyl bedeuten, in 80 - 100 %igem (V/V) Ethanol, wobei die Oxazaphosphorin-Konzentration 10 - 70 Prozent (G/V) beträgt, ausgenommen eine 50 %ige (G/G) Lösung von Cyclophosphamid in 96 %igem (V/V) Ethanol, dadurch gekennzeichnet,
daß man Oxazaphosphorine der Formel I bei einer Temperatur zwischen 15 und 40 °C in 80 - 100 %igem (V/V) wässrigen Ethanol, gegebenenfalls unter Rühren oder Bewegung des Lösungsmittels, auflöst, wobei auf 100 ml des Lösungsmittels 10 bis 150 g Oxazaphosphorln verwendet werden.

3. Lösungen nach Anspruch 1, dadurch gekennzeichnet, daß das Oxazaphosphorin Cyclophosphamid und/oder Ifosfamid ist.

## Claims

1. Solutions of oxazaphosphorins corresponding to the following general formula

* Prozent V/V (Prozentgehalt Volumen in Volumen) bedeutet die Anzahl Milliliter einer Substanz in 100 Milliliter Endprodukt

** Prozent G/V (Prozentgehalt Gewicht in Volumen) bedeutet die Anzahl Gramm einer Substanz in 100 Milliliter Endprodukt.

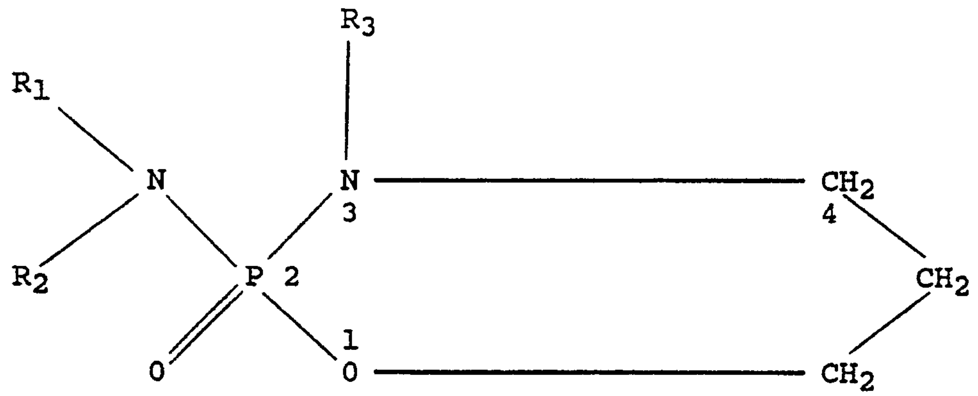

wherein $R_1$, $R_2$ and $R_3$ are identical or different and denote hydrogen, methyl, ethyl, 2-chloroethyl or 2-methane sulphonyloxyethyl and at least two of these groups are 2-chloroethyl and/or 2-methanesulphonyloxyethyl in 80 to 100% (V/V) * ethanol, the oxazaphosphorin concentration being 10 - 70 percent (W/V) ** , with the exclusion of a 50% (W/W) solution of cyclophosphamide in 96% (V/V) ethanol.

**2.** A process for the preparation of solutions of oxazaphosphorins corresponding to the following general formula

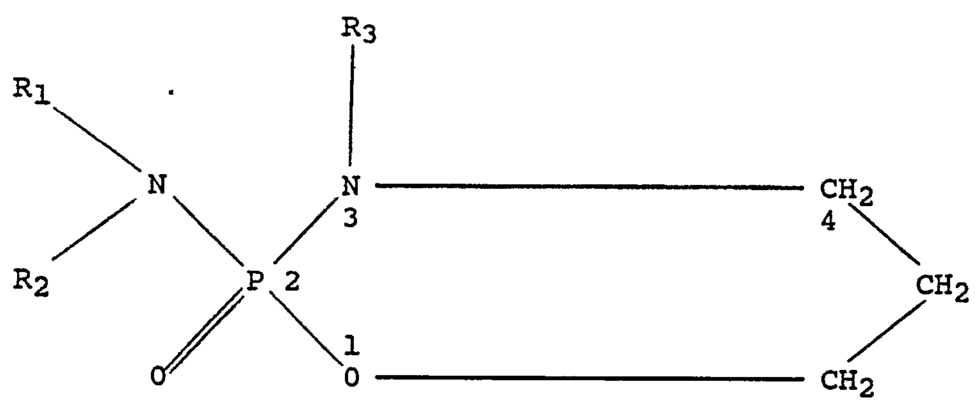

wherein $R_1$, $R_2$ and $R_3$ are identical or different and denote hydrogen, methyl, ethyl, 2-chloroethyl or 2-methanesulphonyloxyethyl in 80 - 100% (V/V) ethanol, the oxazaphosphorin concentration being 10 - 70% (W/V), with the exclusion of a 50% (W/W) solution of cyclophosphamide in 96% (V/V) ethanol, characterised in that oxazaphosphorins corresponding to formula I are dissolved in 80 - 100% (V/V) aqueous ethanol at a temperature from 15 to 40° C, optionally with stirring or agitation of the solvent, from 10 to 150 g of oxazaphosphorin being used per 100 ml of the solvent.

**3.** Solutions according to Claim 1, characterised in that the oxazaphosphorin is cyclophosphamide and/or ifosfamide.

**Revendications**

**1.** Solutions d'oxazaphosphorines de formule générale

* Percent V/V (percentage content volume in volume) denotes the number of millilitres of a substance in 100 millilitres of end product

** Percent W/V (percentage content weight in volume) denotes the number of grams of a substance in 100 millilitres of end product.

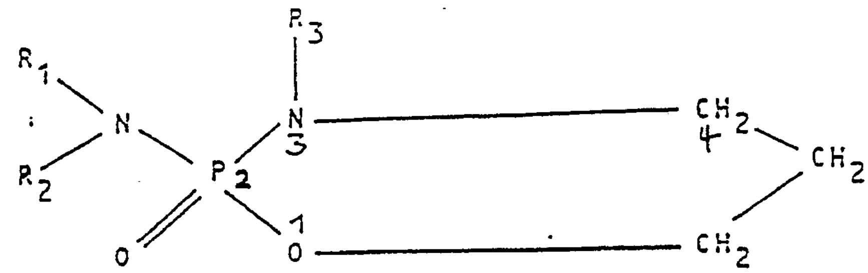

dans laquelle $R_1$, $R_2$ et $R_3$ sont identiques ou différents et représentent chacun un atome d'hydrogene ou un reste méthyle, éthyle, 2-chloréthyle ou 2-méthanesulfonyloxyéthyle, au moins deux d'entre eux étant des restes 2-chloréthyle et/ou 2-méthanesulfonyloxyéthyle, dans de l'éthanol à 80-100% (v/v) *, la concentration d'oxazaphosphorine s'élevant à 10-70° 4 (p/v)**, à l'exception d'une solution à 50% (p/p) de cyclophosphamide dans de l'éthanol à 96% (v/v).

**2.** Procédé de préparation de solutions d'oxazaphosphorines de formule générale

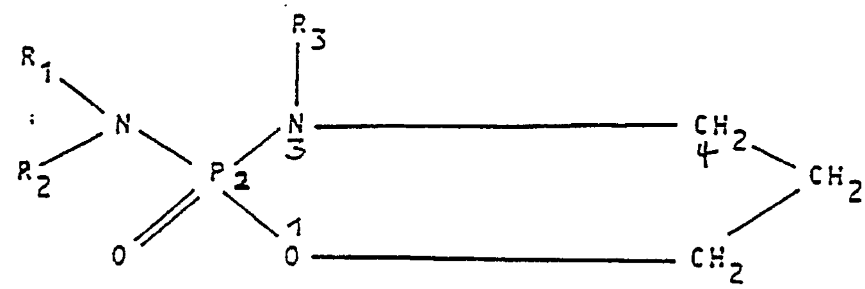

dans laquelle $R_1$, $R_2$ et $R_3$ sont identiques ou différents et représentent chacun un atome d'hydrogène ou un reste méthyle, éthyle, 2-chloréthyle ou 2-méthanesulfonyloxyéthyle, dans de l'éthanol à 80-100% (v/v), la concentration d'oxazaphosphorine s'élevant à 10-70% (p/v), à l'exception d'une solution à 50% (p/p) de cyclophosphamide dans de l'éthanol à 96% (v/v), caractérisé en se qu'on dissout des oxazaphosphorines de formule I, à une température comprise entre 15 et 40° C, dans de l'éthanol aqueux à 80-100% (v/v), éventuellement sous agitation ou mouvement du solvant, en utilisant 10 à 150 g d'oxazaphosphorine pour 100 ml du solvant.

**3.** Solutions selon la revendication 1, caractérisées en ce que l'oxazaphosphorine est le cyclophosphamide et/ou l'ifosfamide.

* % v/v (pourcentage en volume par volume) indique le nombre de millilitres d'une substance dans 100 ml de produit final.

** % p/v (pourcentage en poids par volume) indique le nombre de grammes d'une substance dans 100 ml de produit final.